(19) **Europäisches Patentamt / European Patent Office / Office européen des brevets**

(11) **EP 4 682 262 A1**

(12) # EUROPEAN PATENT APPLICATION
published in accordance with Art. 153(4) EPC

(43) Date of publication:
**21.01.2026 Bulletin 2026/04**

(21) Application number: **24774864.3**

(22) Date of filing: **15.03.2024**

(51) International Patent Classification (IPC):
***C12P 13/02*** *(2006.01)*

(52) Cooperative Patent Classification (CPC):
**C12P 13/02**

(86) International application number:
**PCT/JP2024/010263**

(87) International publication number:
**WO 2024/195728 (26.09.2024 Gazette 2024/39)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **17.03.2023 JP 2023043350**
**17.03.2023 JP 2023043449**

(71) Applicant: **Mitsubishi Chemical Corporation
Tokyo 100-8251 (JP)**

(72) Inventors:
• **YAMAGUCHI Takafumi
Tokyo 100-8251 (JP)**
• **MORI Yasuharu
Tokyo 100-8251 (JP)**

(74) Representative: **Hoffmann Eitle
Patent- und Rechtsanwälte PartmbB
Arabellastraße 30
81925 München (DE)**

(54) **METHOD FOR PRODUCING AMIDE COMPOUND**

(57) A method for producing an amide compound from a nitrile compound in the presence of a biocatalyst having nitrile hydratase activity includes, at least in the presence of air, when a liquid composition of a reaction solution is such that a concentration of the nitrile compound in a gas phase portion of a reactor containing the reaction solution is in an explosion range, performing a hydration reaction under a condition that an oxygen concentration of the gas phase portion of the reactor is 10% by volume or less. The nitrile compound is, for example, preferably acrylonitrile or methacrylonitrile.

**EP 4 682 262 A1**

## Description

TECHNICAL FIELD

The present invention relates to a method for producing an amide compound from a nitrile compound using a biocatalyst having nitrile hydratase activity.

Priority is claimed on Japanese Patent Application No. 2023-043449 and Japanese Patent Application No. 2023-043350, each filed on March 17, 2023, the contents of which are incorporated herein by reference.

BACKGROUND ART

In recent years, a method for producing a compound using a biocatalyst has been used for producing many compounds because of advantages such as mild reaction conditions, simplification of a reaction process, and high purity of a reaction product due to a small amount of by-products.

In the production of an amide compound, since a nitrile hydratase as an enzyme which converts a nitrile compound into an amide compound has been found, the use of the biocatalyst has been vigorously studied (Patent Documents 1 to 4, and the like). In addition, relevant processes have also been vigorously studied, and a large number of proposals have been made for batchwise reaction, semi-batchwise reaction, multi-stage continuous reaction, pipe reactor, and the like.

For example, Patent Document 5 discloses a method for producing acrylamide using a multi-stage continuous reaction apparatus, in which acrylonitrile is supplied to a plurality of reactors on an upstream side of the reaction, and the reaction solution is allowed to mature without supplying acrylonitrile to a plurality of reactors on a downstream side of the reaction.

In addition, Patent Document 6 discloses a method for producing acrylamide by controlling a concentration fluctuation of acrylonitrile during the reaction, but in the method, the acrylonitrile is reacted for a long time in order to reduce the generation of acrylic acid.

Patent Document 7 discloses a method for producing an amide compound using a dried bacterial microorganism.

Citation List

Patent Documents

Patent Document 1: Japanese Unexamined Patent Application, First Publication No. H11-123098
Patent Document 2: Japanese Unexamined Patent Application, First Publication No. H7-265091
Patent Document 3: Japanese Examined Patent Application, Second Publication No. S56-38118
Patent Document 4: Japanese Unexamined Patent Application, First Publication No. H11-89575
Patent Document 5: PCT International Publication No. WO2015/190067
Patent Document 6: Published Japanese Translation No. 2017-535249 of the PCT International Publication
Patent Document 7: Published Japanese Translation No. 2017-529847 of the PCT International Publication

SUMMARY OF INVENTION

Technical Problem

However, the acrylonitrile has very high toxicity, high flammability, low solubility in water and relatively low polarity, and is easily volatilized. When a highly flammable raw material such as the acrylonitrile is used, some measures are required to prevent ignition of a reaction solution containing the raw material and to ensure safety. For example, it is possible to perform the reaction outside an explosion range by reducing the concentration of the raw material in the reaction solution, which, however, poses a problem that the reaction rate is slow and the productivity is low.

In addition, the amide compound such as the acrylamide, which is a target product, is likely to be polymerized. Effective means to suppress the polymerization involves not only addition of a polymerization inhibitor but also presence of a certain amount of oxygen.

In order to prevent the obtained acrylamide from being polymerized, it is desirable that the oxygen concentration is high to some extent. However, an environment with a high oxygen concentration increases the risk of ignition of acrylonitrile as a volatilized raw material present in the gas phase. Therefore, in order to safely achieve high productivity, it is important to control the gas composition in a gas phase portion in the reactor. However, an appropriate oxygen concentration range for avoiding such a situation has not been known so far.

An object of the present invention is to provide a method for safely producing an amide compound while maintaining high productivity.

Solution to Problem

The present inventors have conducted intensive studies in view of the problems of the related art, and as a result, have found that the reaction can be safely performed by appropriately adjusting an oxygen concentration in a gas phase portion in a reactor. Based on this finding, the present invention has been completed. That is, the present invention relates to the following [1] to [8].

[1] A method for producing an amide compound from a nitrile compound in a presence of a biocatalyst having nitrile hydratase activity, the method comprising:

at least in a presence of air, when a liquid composition of a reaction solution is such that a concentration of the nitrile compound in a gas phase portion of a reactor containing the reaction solution is in an explosion range, performing a hydration reaction under a condition that an oxygen concentration of the gas phase portion of the reactor is 10% by volume or less.

[2] A method for producing an amide compound from a nitrile compound in a presence of a biocatalyst having nitrile hydratase activity,

wherein a concentration of the nitrile compound in a reaction solution for producing the amide compound from the nitrile compound by a hydration reaction satisfies the following formula (1):

$$Y \leq 50X - 35 \ (\% \text{ by mass}) \qquad (1),$$

wherein X represents a concentration (% by mass) of the nitrile compound with respect to a total mass of the reaction solution, and Y represents a concentration (% by mass) of the amide compound with respect to the total mass of the reaction solution.

[3] The method for producing an amide compound according to [1] or [2],
wherein a concentration of the nitrile compound in the reaction solution is the following (1) or (2):

(1) the concentration of the nitrile compound with respect to a total mass of the reaction solution is 1.7% by mass or more, or
(2) the concentration of the nitrile compound with respect to the total mass of the reaction solution is 0.7% by mass or more and less than 1.7% by mass, and $Y \leq 50X - 35$ (% by mass) is satisfied, wherein X represents the concentration of the nitrile compound, and Y represents a concentration (unit: % by mass) of the amide compound with respect to the total mass of the reaction solution.

[4] The method for producing an amide compound according to any one of [1] to [3],
wherein, when a hydration reaction rate in a reaction solution A defined below is denoted by S1 and a hydration reaction rate in a reaction solution B defined below is denoted by S2, the biocatalyst used in the reaction is a biocatalyst in which a reaction rate ratio represented by S1/S2 is 0.2 or more, wherein:

the reaction solution A is a reaction solution at 20°C, containing 47% by mass of the amide compound, 2% by mass of the nitrile compound, and water as a balance with respect to a total mass of the reaction solution, and
the reaction solution B is a reaction solution at 20°C, containing 0% by mass of the amide compound, 2% by mass of the nitrile compound, and water as a balance with respect to a total mass of the reaction solution.

[5] The method for producing an amide compound according to any one of [1] to [4],

wherein a time required for reducing a mass of the nitrile compound added to the reaction solution to be halved by the hydration reaction is within 1.5 hours, or

a time required for reducing a concentration of the nitrile compound contained in the reaction solution to be halved by the hydration reaction is within 1.5 hours.

[6] The method for producing an amide compound according to any one of [1] to [5], wherein the nitrile compound is a compound having a double bond, and is stored in a storage container in which an oxygen concentration in the gas phase portion is 1% to 10% by volume in a presence of a quinone-based polymerization inhibitor.

[7] The method for producing an amide compound according to any one of [1] to [6], wherein the nitrile compound is acrylonitrile or methacrylonitrile.

[8] The method for producing an amide compound according to any one of [1] to [7], wherein the amide compound is acrylamide or methacrylamide.

In addition, in another aspect, the present invention relates to the following [A1] to [A5].

[A1] A method for producing an amide compound by performing a hydration reaction of a nitrile compound in a reaction solution in the presence of a biocatalyst having nitrile hydratase activity, in which an oxygen concentration in a gas phase portion of at least one reactor containing the reaction solution is set to 1% to 10% by volume.

[A2] A method for producing an amide compound by performing a hydration reaction of a nitrile compound in a reaction solution in the presence of a biocatalyst having nitrile hydratase activity, in which, when either of the following condition (1) or (2) is satisfied, an oxygen concentration in a gas phase portion of at least one reactor containing the reaction solution is set to 1% to 10% by volume:

(1) a concentration of the nitrile compound with respect to the total mass of the reaction solution is 1.7% by mass or more]; or

(2) a concentration X of the nitrile compound with respect to the total mass of the reaction solution is 0.7% by mass or more and less than 1.7% by mass, and $Y \leq 50X - 35$ is satisfied, wherein Y represents a concentration (unit: % by mass) of the amide compound with respect to the total mass of the reaction solution.

[A3] The method for producing an amide compound according to [A1] or [A2], in which the nitrile compound supplied to the reaction solution is a compound having a double bond and is stored in a storage container in the presence of a quinone-based polymerization inhibitor, and an oxygen concentration of a gas phase portion of the storage container is 1% to 10% by volume.

[A4] The method for producing an amide compound according to any one of [A1] to [A3], in which the nitrile compound is acrylonitrile or methacrylonitrile.

[A5] The method for producing an amide compound according to any one of [A1] to [A4], in which the amide compound is acrylamide or methacrylamide.

In addition, in another aspect, the present invention relates to the following [B1] to [B5].

[B1] A method for producing an amide compound by performing a hydration reaction of a nitrile compound in a reaction solution Z in the presence of a biocatalyst having nitrile hydratase activity, in which, when the biocatalyst exhibits a hydration reaction rate S1 in a reaction solution A and exhibits a hydration reaction rate S2 in a reaction solution B, a reaction rate ratio represented by S1/S2 is 0.2 times or more, and the hydration reaction is performed at a temperature higher than a flash point of the reaction solution Z under an atmospheric composition in at least one reactor containing the reaction solution Z, wherein:

the reaction solution A is a reaction solution at 20°C, containing 47% by mass of the amide compound, 2% by mass of the nitrile compound, and water as a balance with respect to the total mass, and
the reaction solution B is a reaction solution at 20°C, containing 0% by mass of the amide compound, 2% by mass of the nitrile compound, and water as a balance with respect to the total mass.

[B2] The method for producing an amide compound according to [B1], in which a time required for reducing a mass of the nitrile compound added to the reaction solution Z to be halved by the hydration reaction is within 1.5 hours, or a time required for reducing a concentration of the nitrile compound contained in the reaction solution Z to be halved by the hydration reaction is within 1.5 hours.

[B3] The method for producing an amide compound according to [B1] or [B2], in which the biocatalyst is added to the reaction solution Z as a bacterial cell, and an amount of the bacterial cell added is 0.4 g or less in terms of a dry weight of the bacterial cell per 1 kg of the amide compound produced in the reaction solution Z.

[B4] The method for producing an amide compound according to any one of [B1] to [B3], in which the nitrile compound is acrylonitrile or methacrylonitrile.

[B5] The method for producing an amide compound according to any one of [B1] to [B4], in which the amide compound is acrylamide or methacrylamide. Advantageous Effects of Invention

According to the method of the present invention, it is possible to safely produce an amide compound from a nitrile compound while maintaining high productivity (without lowering the productivity).

DESCRIPTION OF EMBODIMENTS

A first aspect of the present invention is a method for producing an amide compound by performing a hydration reaction of a nitrile compound in a reaction solution in the presence of a biocatalyst having nitrile hydratase activity.

Hereinafter, an example of an embodiment of the present aspect will be described.

(1) Biocatalyst having nitrile hydratase activity

In the present embodiment, an amide compound is produced from a nitrile compound in the presence of a biocatalyst having nitrile hydratase activity. In this process, in order to obtain high productivity, the nitrile compound as a raw material may be present at a high concentration in a reaction solution in at least one reactor.

By allowing the nitrile compound to be present at a high concentration in the reaction solution, the nitrile compound volatilizes, and thus a high concentration of the nitrile compound is also present in a gas phase in the reactor. As a result, there is a high risk of ignition or explosion of the nitrile compound due to influence of oxygen present in the gas phase.

In the present embodiment, it is preferable to reduce such a risk as much as possible by rapidly converting the nitrile compound present in the reaction solution into an amide compound using a catalyst having specific performance described later. The catalyst is, for example, preferably a catalyst having a high reaction rate, and more preferably a biocatalyst in which a decrease in hydration reaction rate of the nitrile compound is small even in a situation where the hydration reaction proceeds and the amide compound is accumulated.

The catalyst having a high reaction rate means that a conversion rate from the nitrile compound to the amide compound per unit time is high.

For example, in the present embodiment, by using a catalyst having specific ability described later, the time required for the concentration of the nitrile compound in the reaction solution to be reduced by 50% at the time when the addition of the nitrile compound is completed or interrupted, or the time required for the concentration of the nitrile compound in the reaction solution to be reduced by 50% at the time when the reaction is started can be set to preferably within 1.5 hours, more preferably within 1.2 hours, more preferably within 1 hour, more preferably within 45 minutes, and still more preferably within 30 minutes.

In addition, the biocatalyst in which the decrease in the hydration reaction rate of the nitrile compound is small even in a situation where the hydration reaction proceeds and the amide compound is accumulated is preferably, for example, a biocatalyst in which hydration reaction rate of the nitrile compound in an aqueous solution containing 47% by mass of the amide compound is 0.2 times or more a hydration reaction rate of the nitrile compound in an aqueous solution containing 0% by mass of the amide compound. It is possible to use a biocatalyst in which the above-described hydration reaction rate is more preferably 0.25 times or more and still more preferably 0.3 times or more.

As such a biocatalyst, a biocatalyst in which nitrile hydratase activity is adjusted by using a gene modification technique as described later can be used, or it can be achieved by adjusting the amount (or concentration) of the catalyst to be used.

In the present embodiment, the nitrile hydratase refers to an enzyme having an ability to hydrate the nitrile compound to generate a corresponding amide compound. The biocatalyst having nitrile hydratase activity may be a nitrile hydratase protein itself, but may be an animal cell, a plant cell, a cell organelle, or a microbial cell body, which contains nitrile hydratase, and a treated product thereof.

Examples of the above-described treated product include animal cells, plant cells, cell organelles, a crushed product obtained by crushing a microbial cell body, or an enzyme (a crude enzyme or a purified enzyme) extracted from a microbial cell body; and a carrier on which animal cells, plant cells, cell organelles, a microbial cell body, or an enzyme itself is immobilized.

In addition, the above-described treated product also includes an animal cell, a plant cell, a cell organelle, or a microbial cell body (also referred to as a "dead cell body") which has lost proliferation ability due to a drug treatment.

Examples of the immobilization method include an inclusion method, a crosslinking method, and a carrier bonding method. The inclusion method is a method of coating with a polymer. The crosslinking method is a method of crosslinking an enzyme with a reagent (polyfunctional crosslinking agent) having two or more functional groups. The carrier bonding method is a method of bonding an enzyme to an insoluble carrier.

Examples of the carrier used for the immobilization (immobilized carrier) include glass beads, silica gel, polyurethane, polyacrylamide, polyvinyl alcohol, carrageenan, alginic acid, agar, and gelatin.

Representative examples of such microorganisms include microorganisms belonging to the genera Rhodococcus, Gordona, Pseudomonas, Pseudonocardia, Geobacillus, Bacillus, Bacteridium, Micrococcus, Brevibacterium, Corynebacterium, Nocardia, Microbacterium, Fusarium, Agrobacterium, Acinetobacter, Xanthobacter, Streptomyces, Rhizobium, Klebsiella, Enterobacter, Erwinia, Pantoea, Candida, Aeromonas, Citrobacter, and Achromobacter, which have the nitrile hydratase activity.

More specific examples thereof include Nocardia sp. N-775 described in Japanese Examined Patent Application, Second Publication No. S56-17918; Rhodococcus rhodochrous J-1 described in Japanese Examined Patent Application, Second Publication No. H06-55148; Rhodococcus rhodochrous NCIMB41164 strain described in PCT International Publication No. WO2005/054456; Klebsiella sp. MCI2609 described in Japanese Unexamined Patent Application, First Publication No. H05-30982; Aeromonas sp. MCI2614 described in Japanese Unexamined Patent Application, First Publication No. H05-30983; Citrobacter freundii MCI2615 described in Japanese Unexamined Patent Application, First Publication No. H05-30984; Agrobacterium rhizogenes IAM13570 and Agrobacterium faciens described in Japanese Unexamined Patent Application, First Publication No. H05-103681; Xanthobacter flavus JCM1204 and Erwinia nigrifluens MAFF 03-01435 described in Japanese Unexamined Patent Application, First Publication No. H05-161495; Enterobacter sp. MCI2707 described in Japanese Unexamined Patent Application, First Publication No. H05-236975; Streptomyces sp. MCI2691 described in Japanese Unexamined Patent Application, First Publication No. H05-236976; Rhizobium sp. MCI2610, Rhizobium sp. MCI2643, Rhizobium loti IAM13588, Rhizobium leguminosarum IAM12609, and Rhizobium meriotii IAM12611 described in Japanese Unexamined Patent Application, First Publication No. H05-236977; Candida guilliermondii NH-2, Pantoea agglomerans NH-3, and Klebsiella pneumoniae subsp. pneumoniae NH-26T2 described in Japanese Unexamined Patent Application, First Publication No. H05-15384; Agrobacterium radiobacter SC-C15-1 described in Japanese Unexamined Patent Application, First Publication No. H06-14786; Bacillus smithii SC-J05-1 described in Japanese Unexamined Patent Application, First Publication No. H07-25494; Pseudonocardia thermophila ATCC19285 described in Japanese Unexamined Patent Application, First Publication No. H08-56684; and Pseudonocardia thermophila JCM3095 described in Japanese Unexamined Patent Application, First Publication No. H09-275978.

The Rhodococcus rhodochrous J-1 strain described in Japanese Examined Patent Application, Second Publication No. H06-55148 was deposited on September 18, 1987 under the accession number "FERM BP-1478" at the Patent Organism Depositary, National Institute of Technology and Evaluation (Chuo 6, 1-1-1 Higashi, Tsukuba-shi, Ibaraki-ken (hereinafter the same in this specification)).

The Rhodococcus rhodochrous NCIMB41164 strain described in PCT International Publication No. WO2005/054456 was deposited on March 5, 2003 under the accession number "NCIMB41164" at the National Collection of Industrial, Food and Marine Bacteria, Ltd. (NCIMB) (NCIMB Ltd Ferguson Building Craibstone Estate Bucksburn Aberdeen AB21 9YA).

The Pseudonocardia thermophila JCM3095 described in Japanese Unexamined Patent Application, First Publication No. H09-275978 was deposited on February 7, 1996 under the accession number "FERM BP-5785" at the National Institute of Technology and Evaluation, Patent Organism Depositary (1-1-1 Central 6, Higashi, Tsukuba, Ibaraki Prefecture).

In the present embodiment, one kind of microorganism having a desired characteristic, selected from the above-described microorganisms, can be used alone, or two or more kinds thereof can be used in combination.

A gene encoding the nitrile hydratase can be introduced into and expressed in a microbial cell by a general molecular biological method (for these molecular biological methods, see Sambrook, Fritsch, and Maniatis, "Molecular Cloning: A Laboratory Manual" 2nd Edition (1989), Cold Spring Harbor Laboratory Press). That is, in the present embodiment, an enzyme obtained by expressing a nucleic acid encoding a natural nitrile hydratase (wild type) or a mutant thereof (improved type) in the microbial cell can also be used.

In the present embodiment, one kind selected from the above-described enzymes can be used alone, or two or more kinds thereof can be used in combination.

An amino acid sequence of the wild-type nitrile hydratase is disclosed in the NCBI database such as GenBank (http://www.ncbi.nlm.nih.gov/).

For example, Accession No. of an α-subunit derived from Rhodococcus rhodochrous J1 (FERM BP-1478) is "P21219", and Accession No. of a β-subunit is "P21220". In addition, Accession No. of an α-subunit derived from Rhodococcus rhodochrous M8 (SU1731814) is "ATT79340", and Accession No. of a β-subunit is "AAT79339". Furthermore, Accession No. of an α-subunit derived from Pseudomonas thermophila JCM3095 is "1IREA", and Accession No. of a β-subunit is "1IREB".

Examples of a transformant into which the wild-type nitrile hydratase gene has been introduced include Escherichia coli MT10770 (FERM P-14756) (Japanese Unexamined Patent Application, First Publication No. H8-266277) transformed with a nitrile hydratase of the genus Achromobacter; Escherichia coli MT10822 (FERM BP-5785) (Japanese Unexamined Patent Application, First Publication No. H9-275978) transformed with a nitrile hydratase of the genus Pseudonocardia; and a microorganism transformed with a nitrile hydratase of the Rhodococcus rhodochrous species (Japanese Unexamined Patent Application, First Publication No. H4-211379), but the transformant is not limited thereto.

The improved (mutant)-type nitrile hydratase in which the wild-type nitrile hydratase is subjected to amino acid substitution has been known (Japanese Unexamined Patent Application, First Publication No. 2010-172295, Japanese Unexamined Patent Application, First Publication No. 2007-143409, Japanese Unexamined Patent Application, First Publication No. 2007-043910, Japanese Unexamined Patent Application, First Publication No. 2008-253182, Japanese Unexamined Patent Application, First Publication No. 2019-088326, Japanese Unexamined Patent Application, First Publication No. 2019-088327, PCT International Publication No. WO05/116206, PCT International Publication No. WO12/164933, PCT International Publication No. WO12/169203, PCT International Publication No. WO15/186298, and the like).

In the method of the present embodiment, a microorganism into which these improved-type nitrile hydratases have been introduced can also be used.

These microorganisms having nitrile hydratase activity or the treated product thereof can be used in an amide synthesis reaction immediately after preparation of the bacterial cell, and can also be stored after the preparation of the bacterial cell and used in the amide synthesis reaction as necessary. A microorganism culture method for preparing the bacterial cell can be appropriately selected depending on the kind of the microorganism. A seed culture may be carried out before the main culture.

The microbial cell body having nitrile hydratase activity or the treated product thereof can also be used in a batch reaction or a continuous reaction. In addition, an appropriate reaction form such as a fluidized bed, a fixed bed, or a suspension bed can be selected. In this process, a catalyst temperature in the reaction solution is not particularly limited as long as it does not hinder the mixing of the aqueous medium and the nitrile compound.

With respect to a method for measuring a reaction rate of the biocatalyst, which indicates whether or not the decrease in hydration reaction rate of the nitrile compound is small even in a situation where the amide compound is accumulated by the biocatalyst, an example is described below. In this context, it is assumed that acrylonitrile is used as the nitrile compound and acrylamide is used as the amide compound.

A 100 mL aqueous solution containing 2% by mass of acrylonitrile and 0% by mass of acrylamide, and a 100 mL aqueous solution containing 2% by mass of acrylonitrile and 47% by mass of acrylamide are prepared. A catalyst solution containing a predetermined amount of the catalyst is added thereto to initiate a reaction.

After starting the reaction, sampling is performed a plurality of times at certain time intervals. A sampling time can be, for example, 1 minute to 40 minutes, preferably 2 minutes to 30 minutes and more preferably 3 minutes to 20 minutes. The sample solution is filtered through a filter, phosphoric acid is added thereto, and the reaction is stopped. Thereafter, concentrations of acrylonitrile and acrylamide present in the reaction solution are measured by gas chromatography or liquid chromatography. From the obtained values, a conversion rate from the nitrile compound to the amide compound is calculated, and a rate ratio thereof may be determined.

(2) Nitrile compound

The nitrile compound used as a raw material in the production method according to the present embodiment is not particularly limited as long as it is a compound which is converted into an amide compound by the catalyst having nitrile hydratase activity. Examples thereof include an aliphatic saturated nitrile such as acetonitrile, propionitrile, succinonitrile, and adiponitrile; an aliphatic unsaturated nitrile such as acrylonitrile and methacrylonitrile; an aromatic nitrile such as benzonitrile and phthalonitrile; and a heterocyclic nitrile such as nicotinonitrile. The nitrile compound in the present embodiment is preferably a C2 to C4 nitrile compound such as acetonitrile, propionitrile, acrylonitrile, methacrylonitrile, n-butyronitrile, and isobutyronitrile; and particularly acrylonitrile, methacrylonitrile, are acetonitrile suitable for achieving the effect of the present embodiment.

When a polymerizable nitrile compound such as (meth)acrylonitrile is used, it is preferable to add a polymerization inhibitor. Examples of the polymerization inhibitor include catechols, quinones, benzoquinones, stable radicals such as diphenylpicrylhydrazyl, hindered amines, and phenothiazines. Among the above, quinones are preferable, hydroquinones are more preferable, and methoxyhydroquinone or the like is still more preferable.

An amount of the polymerization inhibitor to be added is not particularly limited, and can be appropriately selected according to the kind of the nitrile compound or the polymerization inhibitor, the storage conditions, and the like. For example, when quinones are used, an amount of the quinones to be added is 1 to 1,000 ppm, preferably 10 to 100 ppm with respect to the amount of the acrylonitrile.

When quinones are used as the polymerization inhibitor, the polymerization inhibition effect can be sufficiently exhibited by the presence of oxygen.

Therefore, as the nitrile compound used in the present invention, it is preferable to use a nitrile compound stored in the presence of dissolved oxygen and a quinone-based polymerization inhibitor. In order to have dissolved oxygen present in the solution, it is preferable that the nitrile compound is stored in a solution containing a quinone-based polymerization inhibitor in the solution, and the solution is stored in a state in which 1% to 10% by volume of oxygen is present in a gas phase portion of the storage container.

(3) Raw water

Water used as a raw material (raw water) is used in the hydration reaction with acrylonitrile when producing acrylamide. Examples of the water include pure water and an aqueous solution in which an acid, a salt, or the like is dissolved in water. Examples of the acid include phosphoric acid, acetic acid, citric acid, boric acid, acrylic acid, and formic acid. Examples of the salt include a sodium salt, a potassium salt, and an ammonium salt of the above-described acid. Specific examples of the water are not particularly limited, but include water such as pure water, ultrapure water, and tap water; and buffer solutions such as a Tris buffer solution, a phosphate buffer solution, an acetate buffer solution, a citrate buffer solution, and a borate buffer solution. A pH (20°C) of the raw water is preferably 5 to 9. The raw water may contain dissolved oxygen. Since the dissolved oxygen exhibits an effect of suppressing the polymerization of the nitrile compound which is a raw material, or the amide compound which is a product, it is not necessary to remove the dissolved oxygen by means such as aeration.

(4) Production of amide compound from nitrile compound using biocatalyst

The method of producing the amide compound from the nitrile compound using the biocatalyst having nitrile hydratase activity according to the present embodiment may be any method such as a batchwise reaction, a semi-batchwise reaction, a multi-stage continuous reaction, or a reaction using a pipe reactor.
The batchwise reaction or the semi-batchwise reaction has an advantage that the high-risk time can be limited to the initial stage of the reaction, particularly in the latter half, due to a significant decrease in vapor concentration of the nitrile compound.
The multi-stage continuous reaction is a reaction in which, using a device having a plurality of continuous reactors, raw materials (including the nitrile compound, the water (as a raw material), and the biocatalyst) are continuously or intermittently supplied to a reactor, and a reaction mixture (hereinafter, also referred to as "reaction solution") in the reactor is continuously or intermittently taken out without being completely withdrawn, and the reaction is performed while transferring the reaction mixture to the next reactor.
In the multi-stage continuous reaction, a plurality of reactors are used, so that there is an advantage that a highly dangerous region (for example, a reactor having a risk of ignition and explosion) can be limited.
The apparatus used in the multi-stage continuous reaction includes two or more reactors connected in series, and produces, in each reactor, the amide compound from the nitrile compound and water by a continuous reaction using the biocatalyst. More specifically, for example, the following method can be used. In the continuous reaction apparatus, the raw materials are added to a reactor located at the most upstream position and a reactor connected to the most upstream reactor to thereby initiate a reaction, and the reaction is allowed to proceed while moving the reaction solution to reactors located sequentially on the downstream side. The reaction solution containing acrylamide, produced from the reactor located most downstream, may be recovered.
The number of reactors is not particularly limited, and can be appropriately selected depending on the reaction conditions and the like. For example, the number of reactors is preferably 2 to 20, more preferably 2 to 12, still more preferably 2 to 10, and even more preferably 2 to 8. The reactors may be present in parallel connection as necessary. The reactors may be independent of each other or may be a large reactor partitioned into the plurality of reactors by a partition wall. With the reactors partitioned by a partition wall, each space partitioned by the partition wall is regarded as one reactor.
A tank for supplying the nitrile compound, the biocatalyst, the raw water, and other auxiliary agents is not limited to one tank at the topmost stream, and may be one tank or a plurality of tanks of two or more tanks. The latter (downstream) tank is used for push cutting and aging in the reaction, and the reaction solution containing a product can be withdrawn from the tank at the lowermost stream (final tank) or the tank present upstream of the lowermost stream.
The number of tanks for supplying the raw material and the number of tanks for aging and the like can be appropriately selected depending on the reaction conditions, the reaction scale, and the like.
The type of the reactor is not particularly limited, and for example, various types of reactors such as a stirring type, a fixed bed type, a fluidized bed type, a moving bed type, a column type, and a tubular type can be used. Among the above, a stirring type in which dispersion and mixing of the raw material can be promoted is preferable. Reactors having different forms can also be connected in combination.
A stirring blade is preferable as a stirrer. The shape of the stirring blade is also not particularly limited, and examples thereof include a paddle, a disc turbine, a propeller, a helical ribbon, an anchor, and a fouler.
As an embodiment of the present aspect, for example, when acrylonitrile is used as a raw material as the nitrile compound to produce acrylamide, both acrylonitrile and acrylamide are compounds which are easy to be polymerized. The polymerization is an exothermic reaction; and once the polymerization starts, it is not easy to stop the polymerization, which may cause serious disasters.

In order to prevent the polymerization in advance, even when the temperature of the entire reaction system is lowered to, for example, 10°C or lower, since a flash point of the acrylonitrile is 0°C, the possibility of ignition and explosion remains. In addition, at a low temperature, a sufficient reaction rate cannot be obtained by the biocatalyst. Furthermore, the produced acrylamide is crystallized and precipitated easily at a low temperature.

On the other hand, when the reaction is performed at a temperature at which a sufficient reaction rate can be obtained, for example, at a temperature of approximately 20°C to 30°C, in order to suppress a volatilized amount of acrylonitrile, the amount of acrylonitrile added to the reaction solution is reduced, and thus sufficient productivity cannot be obtained.

In this context, a preferred embodiment and conditions of the present aspect is described below.

In the reactor in which the hydration reaction of the nitrile compound is performed, it is important to adjust an oxygen concentration in a gas phase portion. More specifically, it is necessary to perform the reaction under a condition that, when a liquid composition of the reaction solution is such that a concentration of the nitrile compound in a gas phase portion of a reactor containing the reaction solution is in an explosion range, an oxygen concentration of the gas phase portion of the reactor is 10% by volume or less. Here, the total capacity of the gas phase portion of the reactor is a capacity obtained by subtracting the volume of the reaction solution (liquid phase) from the total capacity of the reactor.

By setting the oxygen concentration in the gas phase portion of the reactor to 10% by volume or less, it is possible to suppress the ignition or explosion of the nitrile compound as a raw material. The above-described oxygen concentration is preferably 1% to 10% by volume and more preferably 1% to 5% by volume.

In the present specification, the terms "% by volume", "volume%", and "vol%" are interchangeable terms unless otherwise specified.

In addition, in the present specification, the term "explosion range" means a concentration range between an upper limit and a lower limit of the concentration of the nitrile compound when the upper limit of the concentration of the nitrile compound is defined as an upper limit of explosion and the lower limit of the concentration of the nitrile compound is defined as a lower limit of explosion. For example, the "explosion range" when the nitrile compound is acrylonitrile is 3.0% to 17.0% by volume.

In the production method according to the present embodiment, it is preferable that the concentration of the nitrile compound in the reaction solution is a concentration satisfying the following formula (1).

$$Y \leq 50X - 35 \text{ (\% by mass)} \dots (1)$$

wherein X represents a concentration (% by mass) of the nitrile compound with respect to a total mass of the reaction solution, and Y represents a concentration (% by mass) of the amide compound with respect to the total mass of the reaction solution.

In addition, when either of the following condition (1) or (2) is satisfied, the ignition or explosion of the nitrile compound as a raw material can be more reliably suppressed by adjusting the oxygen concentration in the gas phase portion of the reactor to 10% by volume or less.

(1) a concentration of the nitrile compound with respect to the total mass of the reaction solution is 1.7% by mass or more.

(2) a concentration X of the nitrile compound with respect to the total mass of the reaction solution is 0.7% by mass or more and less than 1.7% by mass, and $Y \leq 50X - 35$ (% by mass) is satisfied (here, Y represents a concentration (unit: % by mass) of the amide compound with respect to the total mass of the reaction solution).

Here, the formula (2) is created by obtaining the concentrations of the nitrile compound and the amide compound having a flash point of 40°C or lower, and from the obtained concentration transition. The flash point is measured by a known method such as Cleveland open cup method, Tag closed cup method, and Setaflash method. On the other hand, since it takes time and effort to actually measure all of the broad range of compositions, a method of measuring the concentration of the nitrile compound in the gas phase by changing the temperature with a detection tube or gas chromatography and determining whether or not the composition enters the explosive composition range, or a method of estimating the flash point based on the estimated gas-liquid equilibrium by Wilson's equation or the like may be used.

For example, when acrylonitrile is used as the nitrile compound and acrylamide is produced as the amide compound, in a ternary reaction solution containing water, acrylonitrile, and acrylamide, the flash point of acrylonitrile is higher than 40°C as the concentration of acrylonitrile is less than 0.7% by mass. That is, when the concentration of acrylonitrile in the reaction solution is less than 0.7% by mass, the amide compound can be safely produced even when the temperature of the reaction solution is set to less than 40°C, for example, 20°C to 30°C.

Here, the above-described flash point in the high concentration range is determined by Wilson-RK method for the acrylonitrile vapor pressure of the mixed solution using ASPEN Plus of Aspen Technology Inc. In the low concentration range of 200 ppm or less, the concentration of acrylonitrile in the gas phase portion is obtained by a detection tube. It is preferable to confirm that there is no contradiction between these. Furthermore, the flash point is estimated based on the lower limit of the explosion range of known acrylonitrile explosion range data.

In the above-described reaction solution, when the concentration of acrylonitrile is 1.7% by mass or more, the flash point of acrylonitrile is 40°C or lower regardless of the concentration of acrylamide, and the risk of ignition or explosion of the acrylonitrile is high. Therefore, it is preferable that the oxygen concentration in the gas phase portion is adjusted to 10% by volume or less with respect to the total capacity % of the gas phase portion of the reactor. In this manner, the amide compound can be safely produced.

In the above-described reaction solution, when the concentration of the acrylonitrile is 0.7% by mass or more and less than 1.7% by mass, and $Y \leq 50X - 35$ (X represents the concentration (unit: % by mass) of the nitrile compound in the reaction solution, and Y represents the concentration (unit: % by mass) of the amide compound in the reaction solution) is satisfied, the flash point of the acrylonitrile is lowered to 40°C or lower. Therefore, it is preferable that the oxygen concentration in the gas phase portion is adjusted to 10% by volume or less with respect to the total capacity % of the gas phase portion of the reactor. In this manner, the amide compound can be safely produced.

The method of adjusting the oxygen concentration in the gas phase portion of the reactor is not particularly limited, and for example, the oxygen concentration can be adjusted by allowing an inert gas to flow into the gas phase portion of the reactor.

The type of the inert gas to be allowed to flow is not particularly limited, and for example, nitrogen, argon, neon, helium, krypton, xenon, radon, or the like can be used. These inert gases can be used alone or in combination of two or more kinds thereof. A device or a method for allowing the inert gas to flow is not particularly limited, and can be appropriately selected according to the reaction apparatus, the reaction scale, and the like.

The reaction for which the oxygen concentration is to be adjusted in the gas phase portion is not particularly limited, and the present invention can be applied to any of a batchwise reaction, a semi-batchwise reaction, a multi-stage continuous reaction, or a reaction using a pipe reactor.

The reactor for adjusting the oxygen concentration in the gas phase portion is not particularly limited, and all reactors to be used can be targeted or a part of the reactors to be used can be targeted.

For example, when producing the amide compound by a multi-stage continuous reaction, a plurality of reactors are used. In this case, the oxygen concentration in the gas phase portion may be adjusted in all the reactors, or the oxygen concentration in the gas phase portion may be adjusted in a reactor in which a volatilized amount of the nitrile compound is large, such as a reactor to which the nitrile compound is added. That is, it is also possible to adjust the oxygen concentration in the gas phase portion in the reactor present on the upstream side of the reaction without adjusting the oxygen concentration in the gas phase portion in the reaction tank present on the downstream side of the reaction in which the nitrile compound remaining in the reaction solution (or in the gas phase portion) is reduced.

A water-soluble monocarboxylate having 2 or more carbon atoms can be added to the reaction solution. A timing of adding the water-soluble monocarboxylate is not particularly limited, and the water-soluble monocarboxylate can be added to the reactor located on the most upstream side so that the water-soluble monocarboxylate contained in the reaction solution moves to the downstream side together with the reaction solution, thereby being contained in the reaction solution in each reactor. In addition, the water-soluble monocarboxylate may be added to each reactor before the reaction is started or after the reaction is started.

By adding the water-soluble monocarboxylate having 2 or more carbon atoms, stability of the acrylamide in the reaction solution can be improved.

The water-soluble monocarboxylate may be a saturated monocarboxylate or an unsaturated monocarboxylate. Examples of the saturated carboxylic acid include acetic acid, propionic acid, and n-caproic acid. Examples of the unsaturated carboxylic acid include acrylic acid and methacrylic acid. Examples of the salt include a sodium salt, a potassium salt, and an ammonium salt of the saturated monocarboxylic acid or the unsaturated monocarboxylic acid. These water-soluble monocarboxylates can be each used alone or two or more kinds thereof can be used in combination.

An amount of the water-soluble monocarboxylate to be added is preferably 20 to 5,000 mg/kg with respect to the acrylamide to be produced.

A pH of the reaction in which the acrylonitrile is hydrated to produce acrylamide is preferably 6 to 9 and more preferably 7 to 8.5. A pH measuring method includes an indicator method, a metal electrode method, a glass electrode method, a semiconductor sensor method, and the like; but a measurement by a glass electrode method, which is widely used in industry, is preferable.

A reaction temperature (temperature of the reaction solution) when hydrating the acrylonitrile is not particularly limited; but is preferably 10°C to 50°C, more preferably 15°C to 45°C, and still more preferably 20°C to 40°C. By setting the reaction temperature to be 10°C or higher, the reaction activity of the biocatalyst can be sufficiently increased. In addition, by setting the reaction temperature to be 50°C or lower, inactivation of the biocatalyst can be prevented. In addition, in order to reduce a heat removal load of the reactor, it is preferable that the water or acrylonitrile to be supplied is supplied at a temperature lower than the reaction temperature by 5°C or higher.

In the present embodiment, when a batch reaction is used, the time required for the concentration of the nitrile compound in the reaction solution to be reduced by 50% or less at the start of the reaction is preferably within 1.5 hours, more preferably within 1.2 hours, more preferably within 1 hour, more preferably within 45 minutes, and still more preferably within 30 minutes.

In addition, when a semi-batchwise reaction, a multi-stage continuous reaction, or a pipe reactor is used, the time required for the concentration of the nitrile compound in the reaction solution to be reduced by 50% or less at the time when the addition of the nitrile compound is completed or interrupted, or the time required for the concentration of the nitrile compound in the reaction solution to be reduced by 50% at the time when the reaction is started is preferably within 1.5 hours, more preferably within 1.2 hours, more preferably within 1 hour, more preferably within 45 minutes, and still more preferably within 30 minutes.

In any cases, the reaction proceeds, and the amide compound accumulates in the reaction solution; but even when the amide compound is present in the reaction solution, the time required for the concentration of the nitrile compound in the reaction solution to be reduced by 50% or less at the time when the addition of the nitrile compound is completed or interrupted, or the time required for the concentration of the nitrile compound in the reaction solution to be reduced by 50% at the time when the reaction is started is preferably within 1.5 hours, more preferably within 1.2 hours or less, more preferably within 1 hour, more preferably within 45 minutes, and still more preferably within 30 minutes.

In order to convert the nitrile compound into the amide compound with such a high reaction rate, there are a method of using a large amount of the catalyst, a method of adding the catalyst, a method of using an enzyme having high nitrile hydratase activity or a biocatalyst containing the enzyme, and a method of using a catalyst which is less susceptible to inhibition by the high-concentration amide compound.

The activities of the catalysts are actually compared in terms of units, not in terms of the actual weight of the catalyst. On the other hand, it is advantageous that the actual amount of addition is small from the viewpoint of catalyst removal after the completion of the reaction.

Therefore, it is preferable to use a catalyst having high activity and being less susceptible to inhibition by the amide compound. More specifically, it is preferable to produce the amide compound with a catalytic amount of preferably 2.0 g or less, more preferably 1.0 g or less, more preferably 0.50 g or less, more preferably 0.40 g or less, more preferably 0.35 g or less, and still more preferably 0.30 g or less per 1 kg of the amide compound.

When the addition of the nitrile compound is interrupted, it is preferable to take an interval of 1.5 hours or longer, preferably 1 hour or longer, and more preferably 45 minutes or longer until the addition of the nitrile compound is resumed. This is because the concentration of the nitrile compound in the reaction solution can be sufficiently reduced.

After the addition of the nitrile compound is completed, it is preferable to continue the reaction for 1.5 hours or longer, preferably 1 hour or longer, and more preferably 45 minutes or longer. Likwwise, this is also because the concentration of the nitrile compound in the reaction solution can be sufficiently reduced.

The total amount of the acrylonitrile to be added is determined by the final concentration of the acrylamide; and the final concentration of the acrylamide is 30% by mass or more, preferably 40% by mass or more, and more preferably 45% by mass or more.

The reaction can be performed in a homogeneous system by carrying out the reaction at a maximum concentration of 7% or less, which is a saturation solubility of the acrylonitrile in water; but the present invention is not limited thereto.

When the supply of the acrylonitrile is terminated, the concentration of the acrylonitrile in water decreases from that point. When the decrease is rapid, volatilization of the acrylonitrile is suppressed, and the risk of ignition and explosion of the acrylonitrile in the gas phase is reduced as well.

In the case of a batchwise reaction or semi-batchwise reaction, the above effect is obtained during the reaction aging time in the latter half of the reaction. In the case of a continuous reaction, the above effect is obtained in a downstream portion in the reaction apparatus, where the reaction aging is performed. When the decrease in concentration of the acrylonitrile is rapid, the danger region or the danger time is to be more limited.

For example, a flash point of an aqueous solution containing 48% acrylamide and 2% acrylonitrile in the atmosphere is 37°C, but a flash point of water containing 49% acrylamide and 1% acrylonitrile rises sharply to 56°C, and thus the safety also improves.

A partial pressure of the acrylonitrile is also a value in water at 25°C, but it is halved from 3.8 hectopascals at 2% acrylonitrile to 1.9 hectopascals at 1% acrylonitrile, so that loss or the like is also reduced.

The atmosphere refers to the earth's atmosphere of standard composition including approximately 21% of oxygen.

As an embodiment of the present aspect, in a method of producing an amide compound by performing a hydration reaction of a nitrile compound in a reaction solution (hereinafter, also referred to as "reaction solution Z") in the presence of a biocatalyst having nitrile hydratase activity, the biocatalyst includes, for example, an enzyme having nitrile hydratase activity, and can be added to the reaction solution Z as a living microorganism or as a resting microorganism treated with a drug so as not to lose the enzyme activity.

The above-described biocatalyst preferably exhibits the following properties when the biocatalyst is added in any predetermined amount to the following reaction solution A or the following reaction solution B. That is, when a hydration reaction rate in the following reaction solution A is S1 and a hydration reaction rate in the following reaction solution B is S2, it is preferable that a reaction rate ratio represented by S1/S2 is 0.2 or more. The above-described reaction rate ratio is more preferably 0.3 or more, 0.4 or more, 0.5 or more, and 0.6 or more in this order.

Specific examples of such a biocatalyst include J1 strain used in Examples described later.

[Reaction solution A] a reaction solution at 20°C (which may be 19°C to 21°C), containing 47% by mass (which may be 46% to 48% by mass) of the above-described amide compound, 2.0% by mass (which may be 1.9% to 2.1% by mass) of the above-described nitrile compound, and water as a balance with respect to the total mass of the reaction solution.

[Reaction solution B] a reaction solution at 20°C (which may be 19°C to 21°C), containing 0% by mass of the above-described amide compound, 2.0% by mass (which may be 1.9% to 2.1% by mass) of the above-described nitrile compound, and water as a balance with respect to the total mass of the reaction solution.

The hydration reaction rates S1 and S2 in the reaction solutions A and B are values obtained by sampling a part of the reaction solutions A and B at 10 minutes after the reaction initiation point which is a time point when the reaction solutions A and B are begun to be stirred after adding a predetermined amount of the biocatalyst to the reaction solutions A and B, and measuring an amount of the amide compound produced after a reaction time of 10 minutes.

The mass of the water contained in the reaction solutions A and B is the remaining mass obtained by subtracting the mass of the components other than the water from the total mass of the reaction solutions. The reaction solutions A and B may or may not contain an optional component other than the above-described nitrile compound and the above-described amide compound. A pH buffer is preferable as the optional component. A concentration of the pH buffer is, for example, 0.1 mM to 200 mM.

By using a biocatalyst having the reaction rate ratio of 0.2 or more, the nitrile compound in the above-described reaction solution Z can be rapidly consumed and the flash point of the above-described reaction solution Z can be rapidly increased. As a result, in at least one reactor containing the above-described reaction solution Z, the above-described hydration reaction can be performed with a state in which the temperature quickly transitions to a temperature higher than the flash point of the above-described reaction solution Z under the atmospheric composition.

A suitable nitrile compound fed to the reaction solution Z according to the present embodiment is as described above, and may be one kind or two or more kinds; and it is preferable that the nitrile compound is at least one of acrylonitrile or methacrylonitrile.

The amide compound produced by the reaction solution Z according to the present embodiment preferably has an unsaturated bond, and is preferably at least one of acrylamide or methacrylamide.

An amount of the nitrile compound to be fed (added) to the reaction solution Z can be, for example, 0.10% to 50% by mass, preferably 1.0% to 45% by mass, more preferably 2.0% to 40% by mass, and still more preferably 2.0% to 37% by mass with respect to the total mass of the reaction solution Z contained in the predetermined reactor. Here, the mass of the nitrile compound fed is included in the total mass of the reaction solution Z.

When the amount is equal to or more than the lower limit value of the above-described range, production efficiency of the amide compound increases, and the concentration of the product amide compound increaseas.

In addition, when the amount is equal to or less than the upper limit value of the above-described range, the concentration of the amide compound in the produced aqueous solution is in a preferable range.

A contained amount of the nitrile compound in the reaction solution Z decreases with the progress of the reaction, and is, for example, 0.0001% by mass (1 ppm) to 50% by mass, 2 ppm to 45% by mass, 5 ppm to 40% by mass, or 10 ppm to 37% by mass with respect to the total mass of the reaction solution Z contained in the predetermined reactor.

An amount of the water contained in the reaction solution Z can be, for example, 10.0% to 99.9% by mass, preferably 20.0% to 99.5% by mass, more preferably 30.0% to 80.0% by mass, and still more preferably 40.0% to 70.0% by mass with respect to the total mass of the reaction solution Z contained in the predetermined reactor.

When the amount is equal to or more than the lower limit value of the above-described range, handleability of the produced amide compound is favorable.

In addition, when the amount is equal to or less than the upper limit value of the above-described range, the efficiency of the hydration reaction of the nitrile compound, the efficiency of transportation of the product amide compound, and the like are increased.

The reaction temperature of the reaction solution Z can be set to be higher than the flash point of the reaction solution Z under the atmospheric formulation.

As an example, when performing the reaction in a batch type in which the nitrile compound is fed to the reaction solution Z once, as the hydration reaction of the nitrile compound in the reaction solution Z proceeds and the concentration of the nitrile compound decreases, the flash point of the reaction solution Z increases. That is, in the initial to middle stage of the reaction, the concentration of the nitrile compound is high, and the flash point tends to be low. During the period, the reaction temperature can be raised to a temperature higher than the flash point. In addition, since the flash point of the reaction solution Z increases in the late to end stage of the reaction, the reaction temperature in the period may be lower than the increased flash point.

As another example, when performing the reaction in a non-batch type (continuous type) in which the nitrile compound is continuously or repeatedly fed to the reaction solution Z, the reaction can be performed at a temperature higher than the flash point of the reaction solution Z in a stable period when the supply and consumption of the nitrile compound (production of the amide compound) are balanced and the reaction is stable. In addition, when performing the reaction in a non-batch type by connecting a plurality of reactors in series, the concentration of the nitrile compound in each reactor can be independently controlled, managed, and measured. The reaction can be performed at a temperature higher than the flash point of the reaction solution Z in at least one of the plurality of reactors.

The flash point of the reaction solution Z is measured by a known method such as Cleveland open cup method, Tag closed cup method, and Setaflash method. On the other hand, since it takes time and effort to actually measure all of the broad range of compositions, it is possible to use a method of measuring the concentration of the nitrile compound in the gas phase by changing the temperature with a detection tube or gas chromatography and determining whether or not the composition enters the explosive composition range, or a method of estimating the flash point based on the estimated gas-liquid equilibrium by Wilson's equation or the like.

A period in which the reaction is performed at a temperature higher than the flash point of the reaction solution Z in the total reaction period (for example, 10 minutes to 100 hours) of the reaction solution Z may be 0.1% or more, 1% or more, 5% or more, 10% or more, 20% or more, or 30% or more. The upper limit value of the period is not particularly limited, and as the period is shorter, the safety is higher; but the guideline is 90% or less.

The temperature of the reaction solution Z, that is, the reaction temperature can be controlled, managed, and measured by a temperature control device attached to the reactor containing the reaction solution Z. Examples of the temperature control device include a heater, a Peltier element, and a thermostat.

In the reaction solution Z, the time required for the mass (contained amount) or concentration of the nitrile compound at any point in time to be halved is preferably within 1.5 hours, more preferably within 1.2 hours, more preferably within 1.0 hour, more preferably within 45 minutes, and still more preferably within 30 minutes. As the time required for the half reduction decreases, the flash point of the reaction solution Z can be increased in a shorter time, and thus the safety improves.

Here, the "any point in time" is not particularly limited, but examples thereof include a point in time at which the entire amount of the nitrile compound is fed (added) by a batch method and a point in time at which the reaction is stable by a continuous method. The mass or concentration of the nitrile compound is reduced by the production of the amide compound due to the reaction of nitrile hydratase. The above-described mass or concentration can be measured, for example, by a conventional method such as gas chromatography and liquid chromatography by sampling a small amount of the reaction solution Z.

When the biocatalyst to be added to the reaction solution Z is a bacterial cell, an amount of the bacterial cell added can be grasped in terms of 1 kg of the amide compound produced in the reaction solution Z. When the product is constant, as the amount of the biocatalyst added to the reaction solution Z decreases, the production becomes more economical and the production efficiency improves.

In the present embodiment, the amount of the bacterial cell which is the biocatalyst added to the reaction solution Z is preferably 2.0 g or less, more preferably 1.0 g or less, more preferably 0.50 g or less, more preferably 0.40 g or less, more preferably 0.35 g or less, and still more preferably 0.30 g or less per 1 kg of the amide compound produced in the reaction solution Z.

Here, the amount of the amide compound produced in the reaction solution Z can be calculated by measuring the amount of the amide compound contained in the reaction solution Z at the end of the batch-type reaction by a conventional method such as gas chromatography and liquid chromatography.

Examples

Hereinbelow, the present invention is described in more detail with reference to Examples and Comparative Example, but the present invention is not limited only to Examples described below. In Examples, "%" indicates "% by mass".

[Preparation of biocatalyst]

(1) Preparation of bacterial cell catalyst derived from Rhodococcus rhodochrous J1 strain Pre-culture conditions:

(Culture medium formulation)

Fructose: 2%, Polypepton: 5% (Nippon Pharmaceutical Co., Ltd.), Yeast extract: 0.3% (Oriental Yeast Co., ltd.), $KH_2PO_4$: 0.1%, $K_2HPO_4$: 0.1%, $MgSO_4 \cdot H_2O$: 0.1%, pH: 7
(Culture method)
100 mL of the culture medium was dispensed into a 500 mL Erlenmeyer flask, plugged with a cotton plug, and sterilized in an autoclave at 121°C for 20 minutes. Rhodococcus rhodochrous J1 (FERM BP-1478) was inoculated, and shaken and cultured at 30°C for 48 hours.

Main culture conditions:

(Culture medium formulation)

Initial medium; Yeast extract: 0.2%, $KH_2PO_4$: 0.1%, $K_2HPO_4$: 0.1%, $MgSO_4 \cdot 7H_2O$: 0.1%, $CoCl_2 \cdot 6H_2O$: 0.002%, sulfuric acid: 0.025%, fructose: 2%, urea: 2%, ethanol: 0.4%, Pluronic L61: 0.1% (Asahi Denka Kogyo Co., Ltd.), pH: 7; "Pluronic" is a registered trademark.

Post-addition culture medium; fructose: 20%, ethanol: 5%, sulfuric acid: 6%, pH: 6.5 (Culture method).

2 L of the initial medium was dispensed into a 3 L mini jar fermenter, and sterilized in an autoclave at 121°C for 20 minutes. Here, fructose, ethanol, and urea were separately filtered aseptically (using a 0.45 $\mu$m filter paper manufactured by Advantech Toyo Co., Ltd.), and added to the culture medium. The culture was carried out at an in-tank pressure of 0.098 MPa, a stirring speed of 600 rpm, an aeration amount of 1 vvm, a pH of 7, and a temperature of 30°C; and a small amount of the culture solution was sampled in the middle of the culture. The sample was appropriately diluted with a buffer solution, added to an aqueous solution of acrylonitrile, reacted, and filtered through a disk filter or the like; and the amount of acrylamide (AAM) generated was measured by gas chromatography (Porapak column, column temperature: 210°C, FID detector) to measure activity of nitrile hydratase (reaction rate per unit time and per unit culture solution). The culture was terminated at a point in time at which the nitrile hydratase enzyme activity was no longer increased.

Thereafter, the culture medium was washed with a 50 mM phosphate buffer (pH 7.7) to obtain a bacterial cell suspension having a dry cell weight of 15%. Hereinafter, the obtained bacteria may be referred to as "J1 bacteria".

(2) Comparison of hydration reaction rate in presence and absence of amide compound:

100 mL of a reaction solution (initial pH: 7.0) containing an initial AN concentration of 2%, an initial AAM concentration of 0% or 47%, a phosphoric acid-based pH buffer, and water was prepared and set to 20°C. 0.5 mL of a bacterial cell suspension described later was added to the reaction solution, and stirred to initiate a hydration reaction of AN (20°C). The reaction solution after a reaction time of 10 minutes was sampled, and the amount of AAM produced was measured.

As the above-described bacterial cell suspension, a J1 bacterial cell suspension (bacterial cell concentration (as dry bacteria): 4.11 g/L) was used. (The concentration of the bacterial cell was obtained by dividing the weight of the residue after drying a small amount of the suspension at 125°C for 3 hours by the amount of the sample.)

A reaction rate ratio S1/S2 of a hydration reaction rate S1 of AN in the reaction solution A prepared above, having an initial AAM concentration of 47%, to a hydration reaction rate S2 of AN in the reaction solution B prepared above, having an initial AAM concentration of 0%, was 0.60.

The hydration reaction rates S1 and S2 were calculated from the amount of AAM produced in 10 minutes from the time when the bacterial cell suspensions were added to the above-described reaction solutions A and B and stirred.

<Example 1>

Acrylamide was produced by a semi-batch reaction according to the following procedure.

300 mL of water and 100 mg (1.25 g as slurry) of dried bacterial cells of J1 bacteria as a biocatalyst were charged into a 3 L reactor. The amount of the dried bacterial cells charged was 0.38 g per 1 kg of acrylamide to be produced. Subsequently, 194 g of acrylonitrile (hereinafter, also referred to as "AN"; containing 45 ppm of methoxyhydroquinone) was slowly fed over 3.23 hours. In the reaction solution, the temperature was set to 35°C, and the reaction was carried out by adjusting the pH to 7 with a 1% sodium hydroxide aqueous solution.

At the beginning of the reaction immediately after the start of the feed, the nitrogen gas was continuously flowed into the reactor in the same amount as the volume of the air in the gas phase portion so as to keep the oxygen concentration in the gas phase portion at 10% by volume with respect to the total volume of the gas phase portion, thereby preventing AN in the gas phase portion from igniting. Transition of the concentration of AN was as shown in Table 1.

The AN concentration in the liquid phase and the acrylamide (AAM) concentration in the liquid phase were measured by collecting the reaction solution, appropriately diluting the reaction solution, and then measuring the reaction solution by gas chromatography (column: 1 m of PoraPack-PS (manufactured by Waters Corporation), column temperature: 210°C, carrier gas: helium, FID detector).

The AN concentration in the gas phase was estimated from the liquid phase composition by the Wilson-RK method using ASPEN Plus of Aspen Technology Inc. Furthermore, the low concentration of 300 ppm or less was actually measured using an AN detection tube, and it was confirmed that the value was not significantly deviated. Based on the above, a gas phase flash point was determined depending on whether or not the AN concentration in the gas phase was within the lower limit of the explosion range (3%).

The oxygen concentration in the gas phase was calculated from the amount of inflowing nitrogen.

[Table 1]

| Time elapsed since start of AN feed (left column), time elapsed after AN feed completed (right column) [hr] | | AN concentration in liquid phase [%] | AAM concentration in liquid phase [%] | Gas phase flash point [°C] | AN concentration in gas phase [hPa] | $O_2$ concentration in gas phase [% by volume] | $Y \leq 50X - 35$ |
|---|---|---|---|---|---|---|---|
| 2.90 | -0.33 | 2.20 | 45.98 | 34 | 24.5 | 10 | $45.98 \leq 75$ |
| 3.23 | 0 | 3.19 | 47.89 | 26 | 34.6 | 10 | $47.89 \leq 124.5$ |
| 3.75 | +0.52 | 0.80 | 48.93 | 63 | 9.8 | 21 | $48.93 \geq 5.0$ |
| 4.00 | +0.77 | 0.39 | 49.47 | 95 or higher | 4.9 | 21 | $49.47 \geq -15.5$ |
| 4.50 | +1.27 | 0.10 | 49.87 | 95 or higher | 1.4 | 21 | $49.87 \geq -30.0$ |
| 5.00 | +1.77 | 0.03 | 50.01 | 95 or higher | 0.5 | 21 | $50.01 \geq -33.5$ |

During the AN feed, the gas phase portion was filled with a mixed gas of nitrogen and air, and the oxygen concentration thereof was maintained at 10% by volume. The relationship of Formula ($Y \leq 50X - 35$) was satisfied during the AN feed. The reaction was carried out while keeping the oxygen concentration in the gas phase portion at 10% to thereby enable the reaction to be safely carried out despite the reaction being in a dangerous region in which the reaction solution temperature was higher than the gas phase flash point at 2.90 hours and 3.23 hours during the reaction.

The AN concentration of the reaction solution (liquid phase) was 3.19% at the time of the completion of the feed, and was 0.80% at about 30 minutes after the completion of the feed, which was a reduction of approximately 1/4. The flash point of the gas phase at that point in time (i.e., about 30 minutes after the completion of the feed) was 63°C, and the AN concentration in the gas phase was approximately 9.8 hPa.

The introduction of nitrogen was stopped at a point in time at which the AN concentration in the liquid phase reached 0.80%, the gas phase portion was filled with air, and the reaction was continued.

The AN concentration of the liquid phase decreased to 0.39% or less approximately 0.77 hours after the completion of the feed, the flash point of the gas phase also increased to 95°C or higher, and the AN concentration in the gas phase was 4.9 hPa.

The AN concentration of the liquid phase was reduced to 0.10% or less approximately 1.27 hours after the completion of the feed.

When the reaction of Example 1 was repeated 5 times, all of the reactions could be stably and safely operated.

<Example 2>

AN was hydrated and AAM was produced in the same manner as in Example 1, except that the oxygen concentration in the gas phase portion was changed as described below. That is, at the beginning of the reaction immediately after the start of the feed, the nitrogen gas was continuously flowed into the reactor in an amount three times the volume of the air in the gas phase portion so as to keep the oxygen concentration in the gas phase portion at 5% by volume with respect to the total volume of the reactor, tehreby preventing AN in the gas phase portion from igniting.

The results of transition of the concentration of AN, and the like were as shown in Table 2.

[Table 2]

| Time elapsed since start of AN feed (left column), time elapsed after AN feed completed (right column) [hr] | | AN concentration in liquid phase [%] | AAM concentration in liquid phase [%] | Gas phase flash point [°C] | AN concentration in gas phase [hPa] | O₂ concentration in gas phase [% by volume] | Y≤50X - 35 |
|---|---|---|---|---|---|---|---|
| 2.90 | -0.33 | 2.25 | 45.10 | 33 | 24.8 | 5 | 45.10 ≤ 77.5 |
| 3.23 | 0 | 3.15 | 47.10 | 26 | 33.9 | 5 | 47.10 ≤ 122.5 |
| 3.75 | +0.52 | 0.83 | 48.78 | 63 | 10.2 | 21 | 48.78 ≥ 6.5 |
| 4.00 | +0.77 | 0.42 | 49.31 | 95 or higher | 5.3 | 21 | 49.31 ≥ **-14** |
| 4.50 | +1.27 | 0.05 | 49.78 | 95 or higher | 0.7 | 21 | 49.78 ≥ -32.5 |
| 5.00 | +1.77 | 0.02 | 50.00 | 95 or higher | 0.4 | 21 | 50 ≥ -34 |

During the AN feed, the gas phase portion was filled with a mixed gas of nitrogen and air, and the oxygen concentration thereof was maintained at 5% by volume. The relationship of Formula (Y ≤ 50X - 35) was satisfied during the AN feed. The reaction was carried out while keeping the oxygen concentration in the gas phase portion at 10% so as to enable the reaction to be safely carried out despite the reaction being in a dangerous region in which the reaction solution temperature was higher than the gas phase flash point at 2.90 hours and 3.23 hours duting the reaction.

The AN concentration of the reaction solution (liquid phase) was 3.15% at the time of the completion of the feed, and was 0.83% after about 30 minutes after the completion of the feed, which was a reduction of approximately 1/4. The flash point of the gas phase at the point in time after 30 minutes was 63°C, and the AN concentration in the gas phase was approximately 10.2 hPa. The introduction of nitrogen was stopped at a point in time at which the AN concentration in the liquid phase reached 0.83%, the gas phase portion was filled with air, and the reaction was continued.

The AN concentration of the liquid phase decreased to 0.42% approximately 0.77 hours after the completion of the feed, the flash point of the gas phase also increased to 95°C or higher, and the AN concentration in the gas phase was 5.3 hPa.

The AN concentration of the liquid phase was reduced to 0.1% or less approximately 1.27 hours after the completion of the feed. When the reaction of Example 2 was repeated 5 times, all of the reactions could be stably and safely operated.

As can be seen from the results of Examples 1 and 2, in a stage in which the AN concentration of the reaction solution was relatively high, the AN concentration in the gas phase portion of the reactor tended to be high, and the flash point tended to be low. Therefore, nitrogen gas was allowed to flow into the gas phase portion so as to keep the oxygen concentration thereof at 10% by volume or less, more preferably 5% by volume or less. As a result, it was found that the flash point of the gas phase portion was increased and the safety was improved.

<Example 3>

AAM was produced by a continuous reaction according to the following procedure.

As a reactor, six stirrers (2 L) with jacket cooler were connected in series so that the reaction solution flowed in order from the first tank to the sixth tank. The reactors were connected by an SUS pipe having an inner diameter of 15 mm at a part 1 cm high from the bottom, and the flow rate was adjusted by a control valve. A 4-blade paddle stirrer was attached to each tank. In addition, a pH control device was attached to each tank.

A connecting valve of each reactor was adjusted so that the final concentrations of the AAM aqueous solutions (1.5 L) in each tank were 25%, 38%, 45%, 50%, 50%, and 50% in order from the first tank to the sixth tank. In addition, a biocatalyst as the J1 bacteria was put in the first tank at a rate of 10.1 g/hr (in terms of weight of the dried bacterial cells), and raw water was put in the first tank at a rate of 2050 ml/hr. Furthermore, AN was fed to the first tank at a rate of 548 g/hr, to the second tank at a rate of 443 g/hr, and to the third tank at a rate of 242 g/hr over 30 minutes. The liquid level of each tank was maintained, and the temperature was adjusted to 25°C to 40°C and the pH was adjusted to 7 with a 1% sodium hydroxide aqueous solution.

After the operation for 24 hours, the AN concentration of each tank was measured at a stable state, and the results are shown in Table 3.

[Table 3]

| | Tank 1 | Tank 2 | Tank 3 | Tank 4 | Tank 5 | Tank 6 |
|---|---|---|---|---|---|---|
| AN feed [g/hr] | 548 | 443 | 242 | No | No | No |
| AN concentration in liquid phase [%] | 2.10 | 3.50 | 1.90 | 0.10 | 0.01 | Less than 0.01 |
| AAM concentration in liquid phase [%] | 25.4 | 38.8 | 47.6 | 50 | 50 | 50 |
| AN concentration in gas phase [hPa] | 19.4 | 34.4 | 21.8 | 1.4 | 0.24 | Less **than 0.1** |
| Gas phase flash point [°C] | 36 | 24 | 39 | 95 or higher | 95 or higher | 95 or higher |
| $O_2$ concentration in gas phase [% by volume] | 10 | 10 | 10 | 21 | 21 | 21 |
| $Y \leq 50X - 35$ | $25.4 \leq 70$ | $38.8 \leq 140$ | $47.6 \leq 60$ | $50 \geq$ **-30** | $50 \geq -34.5$ | $50 \geq -34.5$ |

The retention time of each tank was 30 minutes, and the AN concentration rapidly decreased after the fourth tank in which AN was not fed. In addition, since there was no AN fume from the fourth tank and subsequent tanks, and the flash point was sufficiently higher than the reaction temperature from the fourth tank and subsequent tanks, nitrogen gas was not flowed into those tanks. On the other hand, in the first to third tanks, the safety was ensured by mixing the same amount of nitrogen gas with the air in the gas phase portion.

Even when the continuous reaction was carried out by connecting a plurality of reactors as in Example 3 described above, as in Example 1 in which the semi-batch reaction was carried out using a single reactor, the flash point was increased by rapidly reducing the AN concentration fed into the reaction solution in at least one reactor using the biocatalyst (bacterial cell) exhibiting a specific reaction rate ratio, and the nitrogen dilution in the gas phase portion was stopped, so that the air could be circulated. In addition, in the first to third tanks, the hydration reaction could be carried out at a temperature (maximum 40°C) higher than the flash point of the liquid phase under the atmospheric composition.

INDUSTRIAL APPLICABILITY

The present invention is useful in the industrial production of an amide compound such as acrylamide and methacrylamide.

## Claims

1. A method for producing an amide compound from a nitrile compound in a presence of a biocatalyst having nitrile hydratase activity, the method comprising:

   at least in a presence of air, when a liquid composition of a reaction solution is such that a concentration of the nitrile compound in a gas phase portion of a reactor containing the reaction solution is in an explosion range, performing a hydration reaction under a condition that an oxygen concentration of the gas phase portion of the reactor is 10% by volume or less.

2. A method for producing an amide compound from a nitrile compound in a presence of a biocatalyst having nitrile hydratase activity,

wherein a concentration of the nitrile compound in a reaction solution for producing the amide compound from the nitrile compound by a hydration reaction satisfies the following formula (1):

$$Y \leq 50X - 35 \ (\% \text{ by mass}) \qquad (1),$$

wherein X represents a concentration (% by mass) of the nitrile compound with respect to a total mass of the reaction solution, and Y represents a concentration (% by mass) of the amide compound with respect to the total mass of the reaction solution.

3. The method for producing an amide compound according to Claim 1 or 2,
wherein a concentration of the nitrile compound in the reaction solution is (1) or (2) below:

(1) the concentration of the nitrile compound with respect to a total mass of the reaction solution is 1.7% by mass or more, or
(2) the concentration of the nitrile compound with respect to the total mass of the reaction solution is 0.7% by mass or more and less than 1.7% by mass, and $Y \leq 50X - 35$ (% by mass) is satisfied, wherein X represents the concentration of the nitrile compound, and Y represents a concentration (unit: % by mass) of the amide compound with respect to the total mass of the reaction solution.

4. The method for producing an amide compound according to any one of Claims 1 to 3,
wherein, when a hydration reaction rate in a reaction solution A defined below is denoted by S1 and a hydration reaction rate in a reaction solution B defined below is denoted by S2, the biocatalyst used in the reaction is a biocatalyst in which a reaction rate ratio represented by S1/S2 is 0.2 or more, wherein:

the reaction solution A is a reaction solution at 20°C, containing 47% by mass of the amide compound, 2% by mass of the nitrile compound, and water as a balance with respect to a total mass of the reaction solution, and
the reaction solution B is a reaction solution at 20°C, containing 0% by mass of the amide compound, 2% by mass of the nitrile compound, and water as a balance, with respect to a total mass of the reaction solution.

5. The method for producing an amide compound according to any one of Claims 1 to 4,

wherein a time required for reducing a mass of the nitrile compound added to the reaction solution to be halved by the hydration reaction is within 1.5 hours, or
a time required for reducing a concentration of the nitrile compound contained in the reaction solution to be halved by the hydration reaction is within 1.5 hours.

6. The method for producing an amide compound according to any one of Claims 1 to 5,
wherein the nitrile compound is a compound having a double bond, and is stored in a storage container in which an oxygen concentration in the gas phase portion is 1% to 10% by volume in a presence of a quinone-based polymerization inhibitor.

7. The method for producing an amide compound according to any one of Claims 1 to 6,
wherein the nitrile compound is acrylonitrile or methacrylonitrile.

8. The method for producing an amide compound according to any one of Claims 1 to 7,
wherein the amide compound is acrylamide or methacrylamide.

## INTERNATIONAL SEARCH REPORT

| International application No. |
|---|
| **PCT/JP2024/010263** |

**A. CLASSIFICATION OF SUBJECT MATTER**

*C12P 13/02*(2006.01)i
FI:  C12P13/02

According to International Patent Classification (IPC) or to both national classification and IPC

**B. FIELDS SEARCHED**

Minimum documentation searched (classification system followed by classification symbols)

C12P13/02

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| Y | JP 2004-516849 A (LONZA AG) 10 June 2004 (2004-06-10)<br>claims, examples, table 13 | 1-8 |
| Y | WO 2006/093283 A1 (KANEKA CORP.) 08 September 2006 (2006-09-08)<br>paragraphs [0057]-[0060] | 1-8 |
| Y | "混合石油製品の引火点の求め方", ジュンツウネット21 [online], 05 November 2021, [retrieved on 14 May 2024], Internet: <URL: https://www.juntsu.co.jp/qa/qa1706.php>, (JUNTSU NET 21), non-official translation (Method of calculating a flash point for blended petroleum products)<br>table 1, etc. | 3-8 |
| Y | JP 2012-025810 A (TORAY INDUSTRIES, INC.) 09 February 2012 (2012-02-09)<br>paragraphs [0013], [0040] | 6-8 |

☐ Further documents are listed in the continuation of Box C.　　☑ See patent family annex.

| | |
|---|---|
| *　Special categories of cited documents:<br>"A"　document defining the general state of the art which is not considered to be of particular relevance<br>"D"　document cited by the applicant in the international application<br>"E"　earlier application or patent but published on or after the international filing date<br>"L"　document which may throw doubts on priority claim(s) or which is cited to establish the publication date of another citation or other special reason (as specified)<br>"O"　document referring to an oral disclosure, use, exhibition or other means<br>"P"　document published prior to the international filing date but later than the priority date claimed | "T"　later document published after the international filing date or priority date and not in conflict with the application but cited to understand the principle or theory underlying the invention<br>"X"　document of particular relevance; the claimed invention cannot be considered novel or cannot be considered to involve an inventive step when the document is taken alone<br>"Y"　document of particular relevance; the claimed invention cannot be considered to involve an inventive step when the document is combined with one or more other such documents, such combination being obvious to a person skilled in the art<br>"&"　document member of the same patent family |

| Date of the actual completion of the international search | Date of mailing of the international search report |
|---|---|
| **15 May 2024** | **28 May 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**
Information on patent family members

International application No.

**PCT/JP2024/010263**

| Patent document cited in search report | | | Publication date (day/month/year) | Patent family member(s) | | | Publication date (day/month/year) |
|---|---|---|---|---|---|---|---|
| JP | 2004-516849 | A | 10 June 2004 | US | 2004/0142447 | A1 | |
| | | | | claims, examples, table 13 | | | |
| | | | | WO | 2002/055670 | A1 | |
| | | | | EP | 1352050 | A1 | |
| | | | | CN | 1486363 | A | |
| | | | | KR | 10-2008-0099348 | A | |
| WO | 2006/093283 | A1 | 08 September 2006 | US | 2008/0214742 | A1 | |
| | | | | paragraphs [0112]-[0116] | | | |
| | | | | EP | 1857472 | A1 | |
| JP | 2012-025810 | A | 09 February 2012 | (Family: none) | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2023043449 A
- JP 2023043350 A
- JP H11123098 A
- JP H7265091 A
- JP S5638118 B
- JP H1189575 A
- WO 2015190067 A
- JP 2017535249 A
- JP 2017529847 A
- JP S5617918 B
- JP H0655148 B
- WO 2005054456 A
- JP H0530982 A
- JP H0530983 A
- JP H0530984 A
- JP H05103681 A
- JP H05161495 A
- JP H05236975 A
- JP H05236976 A
- JP H05236977 A
- JP H0515384 A
- JP H0614786 A
- JP H0725494 A
- JP H0856684 A
- JP H09275978 A
- SU 1731814
- JP H8266277 A
- JP H9275978 A
- JP H4211379 A
- JP 2010172295 A
- JP 2007143409 A
- JP 2007043910 A
- JP 2008253182 A
- JP 2019088326 A
- JP 2019088327 A
- WO 05116206 A
- WO 12164933 A
- WO 12169203 A
- WO 15186298 A

### Non-patent literature cited in the description

- **SAMBROOK** ; **FRITSCH** ; **MANIATIS**. Molecular Cloning: A Laboratory Manual. Cold Spring Harbor Laboratory Press, 1989